Europäisches Patentamt

European Patent Office

Office européen des brevets

(11) Publication number: **0 126 488**
**B1**

## EUROPEAN PATENT SPECIFICATION

(45) Date of publication of patent specification: **23.04.86**

(21) Application number: **84105839.9**

(22) Date of filing: **22.05.84**

(51) Int. Cl.⁴: **C 07 C 27/12, C 07 C 53/08, C 07 C 49/10, C 07 C 69/14, C 07 C 51/215, C 07 C 45/33, C 07 C 67/00**

(54) Liquid phase oxidation of alkanes.

(30) Priority: **23.05.83 US 497230**

(43) Date of publication of application:
**28.11.84 Bulletin 84/48**

(45) Publication of the grant of the patent:
**23.04.86 Bulletin 86/17**

(84) Designated Contracting States:
**AT BE CH DE FR GB IT LI LU NL SE**

(56) References cited:
**GB-A-2 072 667**
**US-A-4 032 570**

(73) Proprietor: **UNION CARBIDE CORPORATION**
**39 Old Ridgebury Road**
**Danbury Connecticut 06817 (US)**

(72) Inventor: **Kiff, Ben Wilton**
**1414 Grandale Street**
**Lehigh Acres Florida 33936 (US)**
Inventor: **Schreck, David James**
**5226 Sun Valley Drive**
**Cross Lanes West Virginia 25313 (US)**
Inventor: **Woodrow, Philip Travis**
**10516 Leafwood Place**
**Raleigh North Carolina 27612 (US)**
Inventor: **Leung, Siusun Kenneth**
**909 Valley View Drive**
**South Charleston West Virginia 25309 (US)**

(74) Representative: **Wuesthoff, Franz, Dr.-Ing. et al**
**Patentanwälte Wuesthoff -v. Pechmann-Behrens-Goetz Schweigerstrasse 2**
**D-8000 München 90 (DE)**

## Description

### Background of the invention

This invention pertains to the liquid phase oxidation of alkanes and more particularly to the use of oxygen, a cobalt catalyst and a catalyst promoter at elevated temperatures and pressures.

### Background art

Acetic acid is a long-established article of commerce having many uses such as in the manufacture of acetic anhydride and acetate esters, especially vinyl acetate, and as a free acid, solvent or reagent in the production of rubber, plastics, acetate fibers, pharmaceuticals, dyes, insecticides and photographic chemicals.

There are several processes for the manufacture of oxygenated products from hydrocarbons, one route being the liquid phase oxidation of low-molecular weight hydrocarbons, particularly butane.

Butane is now oxidized commercially on a very large scale. The processes which are now in use employ temperatures in the range of 180° to 200°C. These temperatures are required in order to get adequate reaction of the hydrocarbon. To allow for the autogenous pressure of butane at these temperatures, as well as for enough oxidizing gas to provide an oxidizing atmosphere, the pressure must be 800 psig or higher.

Furthermore, the critical temperature of butane is 150°C, considerably below the usual operating temperatures. The oxidation is a liquid-phase reaction and since butane cannot be liquified above 150°C a medium must be provided which will dissolve enough of the butane to undergo an oxidation reaction. Acetic acid is often used as the solvent medium, or alternatively, the mixture of liquid products formed from the oxidation of butane can be used.

Under the conditions described above, butane can be oxidized without the addition of a catalyst, and several commercial acetic acid plants are based on such a process. U.S. 3,904,675 discloses a non-catalytic liquid phase oxidation of butane using small quantities of aldehydes, especially acetaldehyde, to increase productivity and total butane oxidation efficiency. Acetaldehyde also functions to permit the same productivity to be maintained at a lower reaction temperature thereby increasing the production of desirable by-products, such as, methyl ethyl ketone and ethyl acetate which have a higher selling price than acetic acid.

Unfortunately, the use of high levels of acetaldehyde in butane oxidation has a deleterious effect on the economics of the process because acetaldehyde apparently costs more than acetic acid and the improvements in efficiency are offset by this high cost of acetaldehyde.

The technique of using small amounts of soluble metal catalysts has long been known and employed commercially in the large scale oxidation of hydrocarbons, such as, cyclohexane and tetralin. In general, catalyst concentrations in the range of a few parts per million of hydrocarbon promote decomposition of peroxides and increase the reaction rate but not markedly effect the conditions required for the oxidations. In U.S. 3,293,292, describes the liquid phase oxygen oxidation of butane to acetic acid catalyzed by bromine in the presence of catalytic amounts of cobalt and manganese. The liquid phase reaction is carried out in the presence of an inert organic reaction solvent, such as acetic acid. The oxidation temperatures range between 250 and 425°F (121—244°C).

Other results have been reported with a lower temperature process with relatively high concentrations of cobalt catalysts.

In the Canadian 875,856, U.S. 4,032,570 and U.S. 3,923,882 was shown that the simple alkanes can be selectively oxidized by molecular oxygen in the presence of high concentrations of cobalt acetate in acetic acid solvents.

Since the oxidation of butane on a commercial scale produces a plethora of products some with economic value and others with no open market value, it is an object of this invention to provide a process for the liquid phase oxidation of alkanes where the major products are free acids, ketones and esters.

It is a specific object of this invention to provide a process for the liquid phase oxidation of n-butane where the major products are acetic acid, methyl ethyl ketone, and ethyl acetate.

Another object of this invention is to provide a process for the liquid phase oxidation of n-butane which improves raw material efficiency as well as providing the desirable main products.

Other objects will become apparent to those skilled in the art upon a further reading of the specification.

### Summary of the invention

An improved process for the liquid phase oxidation of butane using oxygen, a cobalt catalyst and a catalyst promoter at elevated temperature and pressure has been discovered which comprises carrying out said oxidation with no more than 100 parts of cobalt ion per million parts of the total reaction mixture, up to 10 weight percent of acetaldehyde based on the total reaction mixture as the promoter and an oxygen purity by volume between 19 and 100% at a temperature of 100 to 200°C and super atmospheric pressures of 48,3-62,1 bar (700—900 psig) with preferred pressures of up to 552 bar (800 psig), whereby the efficiency of oxidation is controlled to produce specified products, namely, acetic acid, methyl ethyl ketone, and ethyl acetone.

This discovery is not limited to the liquid phase oxidation of butane but is also applicable to other alkanes having 3 to 16 carbon atoms. When other alkanes are substituted as the substrate of this oxidation the temperature requirements will range from 100 to 200°C and the pressure requirements from 6,9—69 bar (100 to 1000 psig). A preferred pressure for the gas phase oxidation of butane is 48,3 (700) to 62,1 bar (900 psig). The requirements for cobalt in the liquid feed and acetaldehyde are the same as is the oxygen purity, namely between 19 and 100%. The preferred oxygen purity is ~100%. A convenient source for oxygen having a purity of ~19% is air (18—20% by volume). High purity oxygen can be obtained from an oxygen plant where air is liquified and fractionated and (2000 psig) 138 bar cylinder are filled with oxygen having a purity of 99+% by volume. It is understood that oxygen mixtures of any purity between 19 and 100% can be obtained by blending cylinder oxygen with nitrogen or other inert gases. Thus any arbitrary level of oxygen purity can be provided such as 80—90, 50—60, and 30—40 %. However the lower the oxygen purity the lower will be the rate of oxidation of a particular alkane.

Surprisingly it has been found that the interaction between ionic cobalt in low concentrations with acetaldehyde in these alkane oxidation reactions and the interaction between the purity of oxygen used and the level of ionic cobalt used increase the productivity and efficiency of the oxidation reaction above that which can be achieved with the individual effects of cobalt and acetaldehyde.

The significant effect of oxygen dilution on the effect of cobalt as a catalyst in these reactions was also unexpected.

These discoveries overcome previous deficiencies by allowing operation of the oxidation of butane, for example reaction at higher than heretofore achieved productivities with accompanying increases in desired product efficiencies, namely, acetic acid, methyl ethyl ketone, and ethyl acetate. By judicious choice of the acetaldehyde - cobalt concentrations, increased efficiency to one or more of these end products can be controlled while maintaining productivity. This control is in addition to the normally manipulated process variables of temperature, pressure, and reactor residence time.

Exemplary alkanes which can be used in this invention in addition to n-butane include: n-propane, pentane, hexane, hextane, octane, nonane, decane, undecane, dodecane, tridecane, tetradecane, penta-decane and hexadecane. The main products are the corresponding carboxylic acids, dialkyl ketones, alkanols and alkyl esters. Thus for example n-propane affords acetic acid, acetone and isopropyl acetate.

The choice of cobalt compound used to provide the cobalt oxidation catalyst is not narrowly critical. Suitable cobalt compounds include: cobaltous acetate, cobaltous bromide, cobaltous chloride, cobaltous chromate, cobaltous fluoride, cobaltous iodide, cobaltous nitrate, cobaltous oxalate, cobaltous oxide, cobaltous phosphate, cobaltous bicarbonate and cobaltous sulfate.

The concentration of cobalt ion can range from 0.001 to 100 ppm of the total reaction mixtures. A preferred concentration is 0.5 to 5.0 ppm of the total reaction mixture.

The preferred concentration of acetaldehyde is 0.05 to 1.0 weight percent based on the total reaction mixture, although ranges of 0.1 to 10 weight %, 0.2 to 8 weight %, 0.3 to 7 weight %, 0.4 to 6 weight %, and 0.5 to 5 weight % can also be used. While higher levels of acetaldehyde can be used, it is economically undesirable to do so because acetaldehyde is a promoter, not a reactant.

The preferred alkane in the practice of this invention is n-butane. The operating temperature range for this alkane is quite critical. Below 100°C the oxidation reaction cannot be sustained at a commercially feasible rate. Above 200°C selectivity of the oxidation of n-butane to specific end-products is poor and efficiency declines. The preferred reaction temperature range for the oxidation of n-butane in accordance with this invention is 165°C to 200°C and the most preferred range is 168°C to 185°C.

Description of the invention

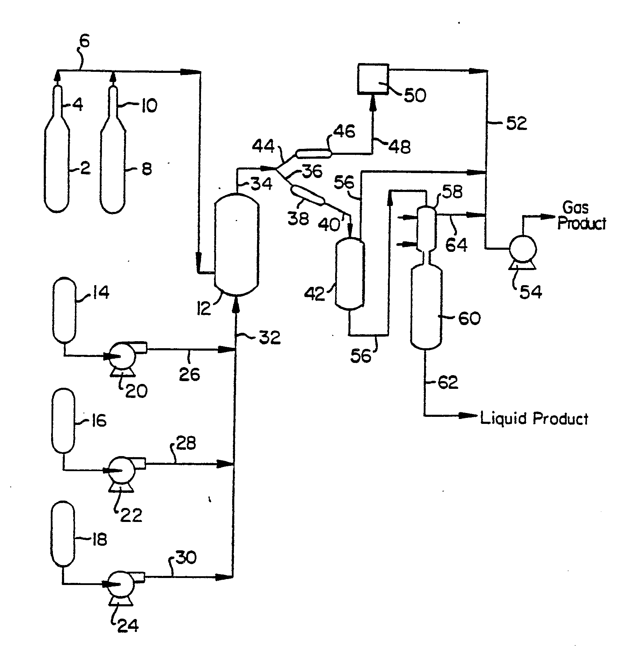

The figure is a flow plan depicting the liquid phase oxidation of butane.

The reactor used for the liquid phase oxidation of normal butane consistent of a one-gallon, type 316 stainless steel autoclave. The autoclave was equipped with a thermowell, gas sparger, internal heating coil and a stirrer having two aggitator blades, one located near the bottom and the other about the middle of the shaft.

Provisions were made for five simultaneous feeds to the reactor, viz., nitrogen, oxygen, acetaldehyde, n-butane and acetic acid. Oxygen and nitrogen were fed from cylinders, the flow rates being measured and controlled by d/p cell-pneumatic controller motor valve arrangements and introduced into the bottom section of the reactor through the gas sparger. The nitrogen was also connected to the reactor outlet and a provision was made to permit feeding the nitrogen either at the bottom of the reactor or at the reactor outlet. This provided a way to measure the effect of oxygen concentration inside the reactor. Flow rates were determined by previous calibration of the controllers. n-Butane and acetic acid were fed and controlled by d/p cell-pneumatic regulated Pulsafeeder pumps. n-Butane was fed to the feed pump from two weighed feed tanks which were pressured to 150 psig with nitrogen. Acetic acid was gravity fed to the pump from a stainless steel feed tank equipped with a calibrated sight glass. Cobalt was added to the reactor by dissolving cobalt acetate in the acetic acid feed. When acetaldehyde was used, it was fed from a calibrated reservoir, pressure to (20 psig) 1,38 bar with nitrogen, with a Milton Roy mini pump and introduced into the system through the acetic acid feed line ahead of the acetic acid d/p cell. Acetaldehyde can also be fed by first mixing with the acetic acid. The organic feeds were fed directly to the bottom of the reactor through a common, heated line.

The crude reaction product, including both the gas and liquid streams, was removed from the top of the reactor through a water cooled 6,35 mm stainless steel line to the top of a 500 cm³ stainless steel pot which acted as both the disengager and liquid level control vessel. The gas stream was removed from the top of the disengager section and let down to atmospheric pressure through a pneumatic controlled motor valve to a dry gas meter. The gas line was tapped at the top of the disengager and a side stream was removed through a 1,6 mm (1/16) inch stainless steel line, a manually controlled valve and a Brooks flow meter to a Hays Magnatherm Oxygen Analyzer, where the gas stream was continually monitored for unreacted oxygen. The liquid level was controlled by a d/p cell pneumatic controlled motor valve through which the crude product, containing n-butane, was let down to atmospheric pressure through a water cooled condenser into a stainless steel product receiver. The product receiver was heated with a heating lamp and the liquid product was kept at 40°C to remove most of the dissolved n-butane. The n-butane was vented off as a gas and fed into the gas vent line which was connected to the dry gas meter.

In a typical run the system was first pressurized to (800 psig) 55,2 bar with nitrogen. After the desired operating pressure had been established and the nitrogen flow stabilized at a rate of (10) 283 l/h (ft³/hr.), the organic feeds were started and fed into the bottom of the autoclave. The feeds were line out according to previously determined rates and continued for about 15 minutes at which time the aggitator was started and controlled at 2000 rpm. Heat was also applied to the reactor by feeding high pressure steam to the internal coil. The temperature was maintained and controlled by a pneumatic control motor valve. These conditions were maintained for 2 hours. Oxygen was then slowly introduced into the reactor through the gas sparger. An exotherm was immediately noticable and as the temperature increased above the control point the steam valve closed. A manual controlled valve was then opened and tap water was injected into the internal coil to cool the reaction. As the reaction cooled to the desired set point the steam valve opened and the pressure generated by the steam flow automatically shut off the water. This system gave excellent temperature control. Once a desired flow of oxygen was established, the nitrogen feed could be switched to the reactor outlet if so desired. After the temperature had equilibrated, the oxygen feed was steadily increased until there was a definite breakthrough of unreacted oxygen as indicated by the oxygen analyzer. The oxygen feed was then slowly decreased until there was 0.5% unreacted oxygen in the gas stream. This was considered to be the maximum oxygen feed for a given set of conditions. These rates were maintained for two hours at which time a run was started. The run consisted of collecting 30 minute liquid and gas samples over a predetermined period of time, usually two hours or more. The liquid samples were removed from the receiver, weighed and mixed well. A small sample was removed and analyzed by gas chromatography. The gas samples were collected as a side stream from the vent side of the gas meter in large weather balloon. The gases were mixed by flexing the walls of the balloon after which a sample was withdrawn into a gas sample bottle. The gas mixture was likewise analyzed by gas chromatography.

Results

The interactions between cobalt, acetaldehyde and oxygen purity can be utilized in several ways. Two key concepts in oxidation chemistry were examined, viz., maintaining productivity (rate) and improving selectivity or increasing productivity and maintaining selectivity. It has been found that either can be accomplished by judicious choice of the above interactions.

Table I
Effect of cobalt and acetaldehyde interaction

| Experiment | 1 | 2 | 3 | 4 |
|---|---|---|---|---|
| Reaction temperature, °C | 180 | 180 | 180 | 180 |
| Acetaldehyde, wt. % of liquid feed | 0 | 1.0 | 0 | 1.0 |
| Cobalt, ppm | 0 | 0 | 9 | 9 |
| Oxygen feed, (SCFH) l/h | (12.6) 357 | (15.1) 427 | (14.0) 396 | (19.2) 543 |

Table II
Effect of nitrogen feed point (oxygen purity)

| Experiment | 1 | 2 | 3 | 4 |
|---|---|---|---|---|
| O$_2$ Feed, SCFH | 19.2 | 19.2 | 19.2 | 19.2 |
| N$_2$ Feed (10 SCFH) | reactor outlet | reactor outlet | sparger | sparger |
| Cobalt concentration, PPM | 0 | 0.5 | 0 | 0.5 |
| Acetaldehyde, wt % of of liquid feed | 0.5 | 0.5 | 0.5 | 0.5 |
| Reaction temperature, °C | 185 | 175 | 195 | 192 |

Table III
Effect on cobalt on temperature and product efficiencies

| Experiment | 1 | 2 | 3 | 4 | 5 |
|---|---|---|---|---|---|
| O$_2$ Feed, SCFH · | 19.2 | 19.2 | 19.2 | 19.2 | 19.2 |
| Cobalt, PPM | 0 | 0.1 | 0.3 | 0.5 | 3 |
| Acetaldehyde, wt.% of liquid feed | 0.5 | 0.5 | 0.5 | 0.5 | •0.5 |
| Reaction temperature °C | 185 | 181 | 177 | 174 | 168 |
| Butane efficiency, % | | | | | |
| Acetic acid | 40.2 | 41.5 | 43.1 | 46.7 | 47.4 |
| Methyl ethyl ketone | 26.5 | 17.0 | 16.8 | 17.3 | 17.5 |
| Ethyl acetate | 10.6 | 9.9 | 9.9 | 9.0 | 8.9 |
| Total | 67.3 | 68.4 | 69.7 | 73.0 | 73.9 |

Table 1 lists the results from 4 runs. Run 1 was a standard oxidation reaction between n-butane and oxygen at 180°C which consumed (12.1 standard cubic feet per hour (SCFH) 342 l/h of oxygen. Run 2 was identical to Run 1 except that it contained 1% acetaldehyde in the feed and it consumed 15.1 SCFH of oxygen, an increase of (2.5 SCFH) 71 l/h over Run 1, a 19.8% increase. Run 3 was identical to Run 1 except that it contained 9 parts per million (ppm) of ionic cobalt and it consumed (14.0 SCFH) 396 l/h of oxygen, an increase of (1.4 SCFH) 39,6 l/h over Run 1, an 11.1% increase. Run 4 was identical to Run 1 except that it contained 1% acetaldehyde and 9 ppm of ionic cobalt and it consumed (19.2 SCFH) 543 l/h, a 52.3% increase in oxygen consumption. The last result was quite unexpected since it had been expected that the individual effects of acetaldehyde and cobalt would have been additive. Thus the expected productivity increase of 19.8% due to 1% acetaldehyde and 11.1% due to 9 parts per million of ionic cobalt would have been a sum of 30.9% productivity increase. However, the observed productivity increase was 52.3%.

Thus an interaction between acetaldehyde and cobalt was observed which was 170% of the expected result. This synergism between acetaldehyde and cobalt was quite unexpected.

In the experimental runs depicted in Table I all of the oxygen was supplied as a mixture of nitrogen and oxygen at a ratio of 0.34 to 0.44.

Table II contains the results of experiments where the oxygen concentration being supplied to the reactor was varied drastically and systematically. Acetaldehyde was present in all runs at a concentration of 0.5% by weight. In runs 3 and 4 nitrogen was co-fed to the reactor with oxygen at a nitrogen to oxygen ratio of 0.34. In runs 1 and 2, pure oxygen was fed to the reactor and nitrogen was added at the outlet of the reactor. (Nitrogen was required for safety reasons). Comparison of Run 1 with Run 3 shows that even though the same amount of oxygen was consumed, the experiment using high purity oxygen reacted at 185°C. With nitrogen diluted oxygen the reaction required 195°. When cobalt was added, at a very low concentration (0.5 ppm), to the nitrogen diluted reaction (Run 4) the reaction temperature

5

dropped to 192°C from 195°C. When the same reaction was repeated using high purity oxygen, the temperature required to consume (19.2 SCFH) 543 l/h was only 175°C. Thus the synergism between the acetaldehyde, cobalt and high purity oxygen resulted in a 20°C temperature drop and only a 3°C drop with diluted oxygen. The ideal for obtaining high oxidation efficiency is low reaction temperatures at high reaction rates.

Since the same amount of oxygen was consumed in each experiment the productivity rate was the same in each run. The benefit of maintaining productivity but lowering temperature is shown in Table III. As the temperature was decreased the selectivity to useful products, viz., methyl ethyl ketone, ethyl acetate and acetic acid, increased from 67.3% to 73.8%. (High purity oxygen was used for these runs).

In the experiment delineated in Table IV note the reduced oxygen consumption (oxygen feed) as well as the poor efficiency. This result shows the deleterious results of high amounts of cobalt. This was unexpected and demonstrates, quantitatively, the criticality of the amount of cobalt used in the claimed process of this invention.

TABLE IV

| Experiment | 1 |
|---|---|
| Reaction temperature | 180 |
| Acetaldehyde, wt. % of liquid feed | 0.5 |
| Cobalt, ppm | 865.5 |
| Oxygen feed, (SCFH) l/h | (4.7) 133 |
| $N_2$, Feed, (SCFH) l/h | (7.6) 215 |
| Butane efficiency, % | |
| Acetic acid | 6.5 |
| Methyl ethyl ketone | 13.9 |
| Ethyl acetate | 20.1 |
| Total | 40.5 |

The invention is further illustrated in the Figure where oxygen is fed from cylinder 2, through stream 4 to line 6 where it joins nitrogen from cylinder 8 passing through line 10 and the gas mixture passing through line 6 is introduced to the bottom section of reactor 12 through a gas sparger. Acetaldehyde, butane and acetic acid are fed from feed tanks 14, 16 and 18 respectively through pumps 20, 22 and 24 respectively and lines 26, 28 and 30 respectively to form stream 32 which delivers the combined feed to the bottom of reactor 12. The crude reaction product, including both gas and liquid streams was removed from the top of reactor 12 through line 34. Liquid products split off into line 36 which passes into water condenser 38 through which the liquid products are further condensed and passed through line 40 into vapor/liquid separator 42.

Gas products from line 34 split into line 44, pass through brine cooler 46 into line 48 through oxygen analyzer 50 into line 52 out through gas meter 54. A gas stream is removed from the top of separator 42 through line 56 which joins line 52 and passes through gas meter 54. The liquid reaction product in separator 42 is removed from the bottom through line 56 under pressure and let down by passing through water condensor 58 into liquid product receiver 60. The liquid product is removed from the bottom of liquid product receiver 60 through line 62. Unreacted butane is vented from water condensor 58 through line 64 which joins line 52 and passes through gas meter 54.

**Claims**

1. A method of liquid phase oxidation of alkanes having 3 to 16 carbons using oxygen, a cobalt catalyst and a catalyst promoter at elevated temperatures and pressures, characterized in that the oxidation is carried out with no more than 100 parts of cobalt ion for a million parts of the total reaction mixture, up to 10.0 weight % of acetaldehyde as the promoter and an oxygen purity by volume of 19 to 100% at a temperature of 100 to 200°C and a pressure of 6,9 to 69 bar whereby the efficiency of oxidation is controlled for the production of carboxylic acids, dialkyl ketones, alkyl esters and alkanols.

2. Method claimed in claim 1 wherein the cobalt ion is introduced as cobalt acetate or is derived from cobaltous oxide.

3. Method claimed in claim 1 or 2 wherein the acetaldehyde concentration is in the range of 0.5 to 10.0.

4. Method claimed in claims 1 to 3 wherein the purity of oxygen is ~100%.

5. Method claimed in claims 1 to 4 wherein the alkane is n-butane or propane.

6. Method claimed in claims 1 to 5 wherein the temperature is 165°C to 200°C.

7. Method claimed in claim 6 wherein the temperature is 168°C to 185°C.

8. Method claimed in claims 1 to 7 wherein the pressure is 48,3—62,1 bar.

**Patentansprüche**

1. Verfahren zur Oxidation von Alkanen mit 3 bis 16 Kohlenstoffatomen in flüssige Phase unter Verwendung von Sauerstoff, einem Cobaltkatalysator und einem Katalysator-Promotor bei erhöhten Temperaturen unter Druck, dadurch gekennzeichnet, daß die Oxidation mit nicht mehr als 100 Teilen Cobaltionen auf 1 Million Teile der gesamten Reaktionsmischung, bis zu 10 Gew.-% Acetaldehyd als Promotor und einem Sauerstoff-Volumenanteil von 19 bis 100 % bei 100 bis 200°C unter einem Druck von 6,9 bis 69 bar durchgeführt wird, wobei die Wirksamkeit der Oxidation im Hinblick auf die Bildung von Carbonsäuren, Dialkylketonen, Alkylestern und Alkanolen eingestellt wird.

2. Verfahren nach Anspruch 1, wobei die Cobaltionen eingeführt werden als Cobaltacetat oder sich ableiten von Cobalt-(II)-oxid.

3. Verfahren nach Anspruch 1 oder 2, worin die Acetaldehyde-konzentration 0,5 bis 10 beträgt.

4. Verfahren nach Anspruch 1 bis 3, worin etwa 100 %-iger Sauerstoff verwendet wird.

5. Verfahren nach Anspruch 1 bis 4, worin das Alkan n-Butan oder Propan ist.

6. Verfahren nach Anspruch 1 bis 5, wobei bei 165 bis 200°C gearbeitet wird.

7. Verfahren nach Anspruch 6, wobei bei 168 bis 185°C gearbeitet wird.

8. Verfahren nach Anspruch 1 bis 7, worin bei einem Druck von 48,3 bis 62,1 bar gearbeitet wird.

**Revendications**

1. Procédé d'oxydation en phase liquide d'alcanes ayant 3 à 16 atomes de carbone, utilisant de l'oxygène, un catalyseur au cobalt et un promoteur de catalyseur à des températures et à des pressions élevées, caractérisé en ce que l'oxydation est conduite avec une quantité ne dépassant pas 100 parties d'ion cobalt par million de parties du mélange réactionnel total, jusqu'à 10,0 % en poids d'acétaldéhyde comme promoteur et une pureté d'oxygène en volume de 19 à 100 % à une température de 100 à 200°C et à une pression de 6,9 à 69 bars, de manière à influencer le rendement d'oxydation pour la production d'acides acboxyliques, de dialkylcétones, d'esters alkyliques et d'alcanols.

2. Procédé suivant la revendication 1, dans lequel l'ion cobalt est introduit sous forme d'acétate de cobalt ou est dérivé de l'oxyde cobalteux.

3. Procédé suivant la revendication 1 ou 2, dans lequel la concentration de l'acétaldéhyde se situe dans l'intervalle de 0,5 à 10,0.

4. Procédé suivant les revendications 1 à 3, dans lequel la pureté de l'oxygène est d'environ 100 %.

5. Procédé suivant les revendications 1 à 4, dans lequel l'alcane est le n-butane ou le propane.

6. Procédé suivant les revendications 1 à 5, dans lequel la température est de 165 à 200°C.

7. Procédé suivant la revendication 6, dans lequel la température est de 168 à 185°C.

8. Procédé suivant les revendications 1 à 7, dans lequel la pression est de 48,3 à 62,1 bars.